## Europäisches Patentamt

(19) **European Patent Office** (11) Publication number: **0 061 912**

**Office européen des brevets** **B1**

(12) **EUROPEAN PATENT SPECIFICATION**

(45) Date of publication of patent specification: **30.09.87** (51) Int. Cl.⁴: **C 07 K 3/02,** C 07 K 15/06, **C 07 K 15/14,** G 01 N 33/53

(21) Application number: **82301605.0**

(22) Date of filing: **26.03.82**

(54) New purified glycoproteins from cow, horse and sheep and use in diagnosing infectious mononucleosis.

(30) Priority: **26.03.81 US 247934**
**27.01.82 US 343235**
**09.03.82 US 356348**

(43) Date of publication of application:
**06.10.82 Bulletin 82/40**

(45) Publication of the grant of the patent:
**30.09.87 Bulletin 87/40**

(84) Designated Contracting States:
**BE CH DE FR GB LI NL SE**

(56) References cited:
**JOURNAL OF IMMUNOLOGY, vol.117, no.3,**
**September 1976, The Williams & Wilkins Co.**
**M.A. FLETCHER et al.: "Immunochemical**
**studies of infectious mononucleosis. V.**
**Isolation and characterization of a glycoprotein**
**from goat erythrocyte membranes", pages**
**722-729**

(73) Proprietor: **UNIVERSITY OF MIAMI**
**Room 230 Ashe Building 100 Memorial Drive**
**Coral Gables Florida 33124 (US)**

(72) Inventor: **Fletcher, Mary Ann**
**10700 Southwest 90th. Avenue.**
**Miami Florida 33176 (US)**

(74) Representative: **De Minvielle-Devaux, Ian**
**Benedict Peter et al**
**CARPMAELS & RANSFORD 43, Bloomsbury**
**Square**
**London WC1A 2RA (GB)**

(56) References cited:
**BIOLOGICAL ABSTRACTS, vol.63, 1977,**
**abstract no. 38581, Philadelphia, Penn. (US)**
**H.J. CALLAHAN: "Preparation of an infectious**
**mononucleosis receptor from sheep**
**crythrocyte stroma"**

**CHEMICAL ABSTRACTS, vol.75, no.17, October**
**25, 1971, page 207, abstract no. 107497t,**
**Columbus, Ohio (US) M.A. FLETCHER et al.:**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**0 061 912**

(58) References cited:
"Immunochemical studies of infectious mononucleosis. I. Isolation and charaterization of heterophile antigens from hemoglobin-free stroma"

CHEMICAL ABSTRACTS, vol.86, no.15, April 11, 1977, page 331, abstract no. 104280h, Columbus, Ohio (US) M.A. FLETCHER et al.:

"Immunochemical studies of infectious mononucleosis. IV. A radioimmunoassay for the detection of infectious mononucleosis heterophile antibody and antigen"

BIOLOGICAL ABSTRACTS, vol.71, 1981, abstract no. 17910, Philadelphia, Penn. (US) B.A. LEVEY et al.: "Latex test for serodiagnosis of infectious mononucleosis"

**0 061 912**

**Description**

This invention relates to purified erythrocyte membrane glycoproteins from mammals such as cow, horse and sheep, a method of using these preparations in immunoassays for the detection of infectious mononucleosis heterophile antibody and the usefulness of these glycoproteins in the preparation of a stable, standardizable reagent for the enumeration of rosetting lymphocytes.

Background of the invention

Attempts at immunological diagnosis or monitoring of the course of infectious mononucleosis (IM) have heretofore emphasized the use of whole erythrocyte fractions. Erythrocytes are known to contain many different membrane proteins, each of which may be reactive with one or more different antibodies [Marchesi, V. T., H. Furthmayr and M. Tomita, *Ann. Rev. Biochem. 45*, 667698 (1976)]. Because whole erythrocytes, or crude erythrocyte fractions, may be agglutinated by antibodies of widely varying characteristics, including many that are unrelated to IM, false positives have been a problem. Avoidance of false positives is clearly important to effective and reliable clinical diagnosis. This invention, in its preferred form, involves homogeneous glycoproteins of cow, horse or sheep, all of which, by virtue of their homogenity, have greater specificity and sensitivity to IM antibodies.

Immunological diagnosis of infectious mononucleosis was first reported in 1932 by Paul and Bunnell in *Amer. J. Med. Sci. 183*, 90—104 (1932). They taught that red blood cells or erythrocytes, from species such as sheep, can be agglutinated by IM heterophile antibodies contained in serum from patients with active infectious mononucleosis. Many assays that detect these heterophile antibodies were subsequently developed, see e.g., U.S. Patents 3,426,123 and 3,708,572.

It was meanwhile reported that preincubation of the patient's serum with guinea pig kidney absorbed or precipitated out the so-called Forssman antibody, a ubiquitous antibody that interferes with the detection of the heterophile antibodies [Davidsohn, I. & P. H. Walker, *Amer. J. Clin. Pathol. 5*, 455—65 (1935)]. Absorption or preincubation of IM serum with guinea pig kidney increased the specificity of the agglutination assay, but required an absorption step before the agglutination reaction was initiated (see, for example, U.S. Patents 3,959,456 and 3,864,467).

Stuart et al. and Beer reported that horse erythrocytes reacted with the heterophile antibodies found in sera of patients with infectious mononucleosis. See *Proc. Soc. Exp. Biol.* 34: 212, 215 (1936) and *J. Clin. Invest.* 15: 591—599.

Bovine erythrocytes were also found to be specific in detecting IM heterophile antibodies. In the early work on red blood cells of this species, Bailey and Raffel [*J. Clin. Invest. 14*, 228—244 (1935)] found that native bovine erythrocytes were not agglutinated by serum containing IM antibodies, but that these erythrocytes did absorb the heterophile antibody and were lysed in the presence of complement. This bovine erythrocyte hemolysin test was later reported to be more specific for heterophile antibody than agglutination tests using horse or sheep erythrocytes, even when a preabsorption step with guinea pig kidney was utilized. [Evans, A. S. et al., *J. Infect. Dis. 132*, 546—554 (1975)]. Evans et al., suggested that bovine erythrocytes contain immunological determinant(s) which bind more specifically to heterophile antibodies than the immunological determinants present in erythrocytes from other species, such as goat, sheep or horse.

These observations on the immunoreactivity of mammalian erythrocytes with IM heterophile antibodies have provided the foundation for the immunochemical diagnosis of infectious mononucleosis by immunoassays using isolated and purified glycoproteins from mammalian erythrocytes.

One method of partial purification employs a hot phenol extract step [Callahan, H. J., *Int. Archs. Allergy Appl. Immun.* 51: 696—708 (1976)], used with material from sheep. This method suffers from the problem that if the stroma contains hemoglobin, the extraction with hot phenol will prevent subtantial removal of the hemoglobin because of denaturation. The present invention encompasses a method of isolating cow, sheep or horse stroma free of hemoglobin, and then subjecting the product stroma to hot ethanol extraction. The resulting glycoprotein product is pure and has a high reactivity with the IM heterophile antibody.

Glycoprotein fractions have been isolated from horse by chloroform methanol extraction. These fractions were closely associated with certain blood group antigens. [Guertler, K. G. Schmid, D. O., Yebou, D. A. and Cleve, H., *Blood Grps. Biochem. Genets.* 9: 41—45 (1978)]. However, the product obtained was not tested for reactivity with IM or other Paul-Bunnell positive antibodies. A close examination of the reported data revealed that the isolated glycoprotein was impure and was hence unsuitable for use in assays as sensitive as radioimmunoassays.

Other investigators have reported glycoproteins isolated from various mammalian species and have obtained "purified" glycoprotein. For example, Hamaguchi and Cleve reported a method of isolating glycoprotein from human, ox, swine, horse and sheep erythrocytes by chloroform-methanol extraction. However, the isolated extract was crude by comparison to the purified glycoprotein of this invention. The serological activity of the Hamaguchi preparation were about 1000-fold less than that of this invention. [See also Fujita, S., Cleve, H., *Biochim. Biophys. Acta* 406: 206—213 (1975); Grant, D. C., Martin, W. G. and Anastassiadis, P. A., *J. Biol. Chem.* 242(17): 3912—3918 (1967); Hamazaki, H., Hotta, K., Konishi, J., *Comp. Biochem. Physiol.* 55 B: 37—44 (1976)].

3

As early as 1971, the present inventor and coworkers [Fletcher, M. A., and Woolfolk, B. J., *J. Immunol.* 107: 842—853 (1971)] found that the partial purification of bovine erythrocytes with 75% ethanol yielded a crude glycoprotein extract which showed high reactivity with heterophile antibodies. The reactivity was based on the result of each of three test methods which measured hemagluttination inhibition, quantitative precipitation and agar gel diffusion.

Later, the present inventor and co-workers [Fletcher et al., *J. Immunol.* 117: 722—729 (1976)] attempted to isolate IM antigen from goat erythrocytes. Again this procedure involves extraction with 75% ethanol, but further involves a lipid extraction step. However, this process is believed to produce a glycoprotein which is contaminated with non-IM antigens.

Several diagnostic tests for IM have been reported that are based on bovine erythrocyte antigens and which employ crude extracts as the purest form of the reactive principle in bovine erythrocytes. For example, see U.S. Patents 3,826,821; 3,840,655; 3,959,456 and 4,228,148.

The applicant has purified the bovine erythrocyte membrane glycoprotein to an essentially homogeneous form. The homogeneity of this bovine glycoprotein was shown in several ways, including polyacrylamide gel electrophoresis in the presence of sodium dodecyl sulfate. The purified form of this bovine glycoprotein for IM detection resulted in at least a ten-fold increase in sensitivity in hemagglutination inhibition tests relative to the results obtained with the crude extract of bovine erythrocytes described by Fletcher et al. in the year 1971. The results obtained with this glycoprotein in partially purified form were reported on March 27, 1980 by the present inventor and co-workers in Levey B. A. et al., *J. Clin. Microbio.* 11, 256—262 (1980).

The present invention involves the isolation of glycoproteins from cow, horse and sheep erythrocytes which glycoproteins react with IM but not with Forssman antibodies. The bovine, horse and sheep erythrocyte glycoproteins of this invention are essentially homogeneous. The IM activity is associated with certain sialoglycoproteins and is completely abolished by neuraminidase treatment. The glycoproteins of this invention carry the antigenic determinant responsible for the reaction with the Paul-Bunnell antibody. This characteristic has made possible the development of certain serological diagnostic tests for IM which have heretofore required intact erythrocytes and complicated, time consuming adsorption procedures. See, for example, Levey et al., *supra*.

An additional receptor property of the horse and sheep glycoproteins isolated here is their ability to interact with peripheral blood lymphocytes forming "E rosettes" *in vitro*. An "E rosette" is a peripheral blood lymphocyte to which three or more sheep red blood cells have become attached. Rosetting is an accepted marker for human T lymphocytes and thymocytes. In this invention the receptor which is responsible for rosetting has been isolated from horse and sheep erythrocyte glycoprotein. Thus, the purified horse or sheep reagents may be useful in the preparation of a standardizable reagent for the enumeration of rosetting lymphocytes.

The homogeneous glycoproteins of this invention and the opportunity they afford for development of sensitive immunoassays may make possible a rapid and useful means of detecting and quantifying antibody to Epstein-Barr virus *per se*, as distinguished from the heterophile antibodies that are detected by test methods of the prior art. The need for such a practical test has been recognized, e.g., in a recent editorial in *Brit. Med. J. 280*, 1153—1154 (1980).

Brief description of the invention

The present invention affords a novel purification procedure for obtaining an essentially homogeneous form of bovine glycoprotein (BGP) from bovine erythrocytes, for obtaining an essentially homogeneous form of horse glycoprotein (HGP) from horse erythrocytes, and for obtaining an essentially homogeneous form of sheep glycoprotein (SGP) from sheep erythrocytes, each of which acts as an antigen in testing for the presence of heterophile antibodies of human infectious mononucleosis.

In its essentially homogeneous form, the BGP of this invention contains essentially no complex glycolipid. The homogeneous BPG of this invention forms substantially a single band upon gel electrophoresis at pH 7.0 when stained with either Coomassie blue or a Schiff reagent containing periodic acid, on a phosphate-buffered polyacrylamide gel in a dilute detergent such as sodium dodecyl sulfate.

The amino acid composition of the essentially homogeneous BGP, in moles per 100 moles of BPG is aspartic acid about 7.2, threonine about 8.0, serine about 7.2, glutamic acid about 16.5, proline about 12.9, glycine about 8.9, alanine about 5.6, valine about 5.4, methionine about 1.2, isoleucine about 6.4, leucine about 9.2, tyrosine about 0.9, phenylalanine about 2.8, histidine about 1.3, lysine about 1.8 and arginine about 4.8. Minor variations principally attributable to differences among individual cows used as sample source will, of course, be observed as those of ordinary skill in the art will readily understand.

The amino acid composition of the purified horse glycoprotein in moles per 100 moles of HGP is as follows: aspartic acid about 8.1, threonine about 10.6, serine about 10.8, glutamic acid about 9.4, proline about 12.3, glycine about 9.2, alanine about 11.3, valine about 4.4, methionine about 0.8, isoleucine about 3.5, leucine about 8.2, tyrosine about 1.1, phenylalanine about 2.9, histidine about 1.2, lysine about 1.3, and arginine about 4.8. The composition of threonine has been corrected for about a five percent loss on hydrolysis and that of serine has been corrected for about a ten percent loss on hydrolysis.

The amino acid composition of the purified sheep glycoprotein in moles per 100 moles of SGP is as follows: aspartic acid about 5.6, threonine about 8.1, serine about 12.9, glutamic acid about 13.0, proline about 11.6, glycine about 7.7, alanine about 9.6, valine about 6.2, methionine about 0.5, isoleucine about 4.6, leucine about 8.3, tyrosine about 1.6, phenylalanine about 1.2, histidine about 1.6, lysine about 3.2, arginine about 4.0, and tryptophan about 0.3.

Carbohydrates account for about twenty-five percent by weight of the homogeneous bovine glycoprotein, about fifty-five percent by weight of the homogeneous horse glycoprotein and about fifty-seven percent by weight of the homogeneous sheep glycoprotein. Protein accounts substantially for the balance in each glycoprotein.

The homogeneous, highly purified erythrocyte glycoproteins of this invention provide stable, standardizable reagents which will keep indefinitely at room temperature without loss of biological activity. These glycoproteins may be attached to inert supports and may also be labelled with tags such as radioactive tags, and used in immunoassays such as radioimmunoassays, for the detection of the Paul-Bunnell antibody and for the detection of certain subpopulations of lymphocytes.

A special embodiment of this invention involves a receptor property of the isolated glycoprotein of sheep and horse erythrocytes which is the ability of this receptor to interact with human T lymphocytes, and thus to inhibit the formation of rosettes. The formation and the enumeration of rosetting lymphocytes required the availability of fresh sheep blood which requires special handling for shipping and storage. In addition, fresh sheep blood has limited shelf life even when stored at 4°C.

Detailed description of the invention

The products described here are purified glycoproteins useful as a diagnostic reagent in the detection of infectious mononucleosis. The product purity achieved by this invention provides a level of antigenic sensitivity and specificity which has not heretofore been possible with other IM diagnostic preparations. The antigens described here were isolated and purified from cow, sheep or horse erythrocytes in a complex series of steps which involved, *inter alia*, ethanol precipitation, phosphocellulose chromatography, lipid solvent extraction and diethyl amino ethyl (DEAE) chromatography. It will be understood that substantially the same purification is used for each species, whether cow, sheep or horse.

In sequence, hemoglobin protein from cow, horse or sheep is first removed from red cell membranes, which are then extracted by a sequential organic solvent treatment which yields a crude GP comprising a 75% ethanol extract. These preliminary steps were first described by the applicant and a co-worker in 1971; see Fletcher et al. cited *supra*.

According to the present invention, the extract obtained with 75% ethanol is further treated chromatographically on a cation-exchange column and then extracted with a series of at least two different lipid solvents to yield a product which contains up to about 10% by weight of complex glycolipid admixed with homogeneous glycoprotein. This product, which is referred to hereinafter as 'partially purified BGP', exhibits a single band at pH 7.0 in a gel electrophoresis test wherein the product is stained with Coomassie blue or a Schiff reagent containing periodic acid, and the gel is phosphate-buffered polyacrylamide gel in a dilute detergent solution, e.g., 0.1% sodium dodecyl sulfate.

In a final series of steps performed according to this invention, the complex glycolipid is substantially entirely removed by binding the product on an anion exchange column and eluting with a high salt buffer. This product exhibits substantially a single band at pH 7.0 in a gel electrophoresis test wherein the product is stained with Coomassie blue or a Schiff reagent containing period acid, and the gel is phosphate-buffered polyacrylamide gel in a dilute detergent solution, e.g., 0.1% sodium dodecyl sulfate.

It has been found that the homogeneous glycoproteins of this invention contain essentially no glycolipid. Homogeneous BGP contains approximately 25% carbohydrate, whereas homogeneous HGP contains approximately 55% carbohydrate, and homogeneous SGP contains approximately 57% carbohydrate. The rest of each glycoprotein is substantially protein.

It has also been found that the glycolipid removed by this series of steps is essentially nonreactive to antibodies which characterize human infectious mononucleosis. Its removal is of special importance to the achievement of a practical immunoassay method, however, because in these methods, one must be able to assume that the label (whether a radioactive isotope, a fluorogen, a chromogen, a luminescent material or another known type of label) is substantially entirely bound to a specific immunochemical reactant. Inadvertent labelling, even of a material inert to all other substances present, renders this assumption invalid and leads to results that are invalid or unable to be reliably interpreted. Moreover, a labelled contaminant, even if non-reactive with the immunochemical entity being assayed for, may well be reactive with another component in a test sample and hence may contribute to the magnitude of readings obtained, thereby disrupting the assay and causing it to give false information. It is therefore essential to the development of successful immunoassays for diagnosis and monitoring of human IM that the complex glycolipid be essentially completely removed from BPG to be used in such immunoassays.

The partially purified BGP referred to above not only exhibits a single band in an electrophoresis test on phosphate-buffered SDS-modified polyacrylamide gel at pH 7.0, buts its composition is markedly different from that of the crude bovine antigen preparation described by applicant and coworker in 1971. The following table compares the molar ratios of constituent sugars present in the crude and partially purified products:

TABLE I

| Sugar | Crude BGP antigen | Partially purified BGP (containing about 10% glycolipid) |
|---|---|---|
| Hexose | 2 | 1.4 |
| Sialic acid | 1 | 1 |
| N-acetylgalactosamine | 0.7 | 0.4 |
| N-acetyl-glucosamine | 1 | 0.5 |

Table II shows the carbohydrate composition of the partially purified BGP, still containing about 10% of complex glycolipid, in micro-moles per milligram of protein:

TABLE II

| Sugar | Micro-mol/mg of protein |
|---|---|
| Galactose | 0.526 |
| Mannose | 0.033 |
| N-acetylglucosamine | 0.191 |
| N-acetylgalactosamine | 0.172 |
| Sialic acid | 0.389 |

The values in Table II, except for that of sialic acid, were obtained by a conventional gas-liquid chromatography technique after first hydrolyzing the sugar at 100°C for 3 hours in 3N HCl. The value for sialic acid was determined conventionally by the alkaline-Ehrlich test and was calculated as N-glycolylneuraminic acid. Table II was reported in Levey, B. A. *et al., J. Clin. Microbio. 11,* 256 (1981).

Table IIa shows the carbohydrate composition of the homogeneous BGP of this invention, as obtained on a different sample taken from a different bovine individual from that on which the Table I and II figures were obtained.

TABLE IIa
Carbohydrate composition of homogeneous BGP

| | Micro-moles/mg glycoprotein | g/100 g glycoprotein |
|---|---|---|
| Polypeptide | 7.163 | 73.30 |
| N-glycolylneuraminic acid | .297 | 9.17 |
| Galactose | .448 | 7.26 |
| N-acetylglucosamine | .322 | 6.54 |
| N-acetylgalactosamine | .161 | 3.27 |
| Mannose | .028 | 0.46 |

The values in Table IIa, except for N-glycolylneuraminic acid and polypeptide content, were obtained by a conventional gas-liquid chromatography technique after first hydrolyzing the sugar at 100°C for about 3 hours in 3N HCl. The value for N-glycolylneuraminic acid was determined conventionally by the alkaline-Ehrlich test.

The homogeneous bovine glycoprotein has approximately 75 weight % of protein and 25 weight % carbohydrate. Minor variations in determinations such as these were found. One variation was between the sample used for Tables I and II, and the sample used for Tables IIa and IIb. Specifically the sample used for Tables I and II had a weight % of protein equal to approximately 77% and a weight % of carbohydrate equal to approximately 23%, whereas the sample used for Tables IIa and IIb had a weight % of protein equal to approximately 73% and a weight % of carbohydrate equal to approximately 27%. As those skilled in the

6

# 0 061 912

biological arts will readily understand, such differences between individual and individual are minor and are to be expected from samples of individual origin.

Since the weight % of protein was approximated from the number of moles of amino acids subjected to amino acid analysis, the observed differences in weight % are also attributable, of course, to different amino acid analyzers used for each sample, and to different buffer systems employed in the amino acid analyzer.

Table IIb compares the molar ratios of constituent sugars present in the partially purified BGP and in the homogeneous BGP.

### TABLE IIb

| Sugar | Partially purified BGP (containing 10% glycolipid) | Homogeneous BGP (with glycolipid removed) |
|---|---|---|
| N-glycolylneuraminic acid | 1 | 1 |
| Galactose | 1.35 | 1.50 |
| N-acetylglucosamine | 0.49 | 1.08 |
| N-acetylgalactosamine | 0.44 | 0.54 |
| Mannose | 0.08 | 0.09 |

Note that the homogeneous BGP of this invention has a substantially higher molar ratio of N-acetylglucosamine to N-glycolylneuraminic acid, indicating that removal of the complex glycolipid from the partially purified BGP significantly changed the carbohydrate composition. It should also be borne in mind that this comparison was made using two different samples obtained from different bovine individuals, and that individual differences among living things to some extent always affect numerical values obtained.

Tables IIc and IId give the carbohydrate composition of homogeneous horse glycoprotein (HGP) and of homogeneous sheep glycoprotein (SGP), respectively.

### TABLE IIc
### Carbohydrate composition of homogeneous horse glycoprotein (HGP)

| Sugar residue | Moles/mg glycoprotein (molar ratio) | g/100 g glycoprotein |
|---|---|---|
| Polypeptide | — | 45.0 |
| N-glycolylneuraminic acid | 0.855 (1.3) | 28.0 |
| Galactose | 0.635 (1) | 11.4 |
| N-acetylgalactosamine | 0.637 (1) | 14.1 |
| N-acetylglucosamine | 0.040 (0.1) | 0.9 |
| Mannose | 0.040 (0.1) | 0.7 |

7

TABLE IId
Carbohydrate composition of homogeneous sheep glycoprotein (SGP)

| Sugar residue | Moles/mg glycoprotein | g/100 g glycoprotein |
|---|---|---|
| Polypeptide | — | 44.0 |
| Sialic acids | 0.541 | 16.7 |
| Galactose | 0.558 | 9.0 |
| N-acetylgalactosamine | 0.676 | 13.7 |
| N-acetylglucosamine | 0.709 | 14.4 |
| Mannose | 0.104 | 1.7 |

The carbohydrate composition of Tables IIc and IId were determined by the methods used for Table IIa.

Table III compares the partially purified BGP with the crude BGP reported by applicant and coworker in 1971, in terms of amino acid composition in moles per 100 moles of total BGP present. These values were determined by hydrolyzing samples in 6N HCl at 100°C in sealed, evacuated tubes for a 24-hour period, chromatographing the product so obtained and analyzing with a Durran 500 automatic amino acid analyzer. No corrections were made for loss due to hydrolysis.

TABLE III

| Amino acid | Crude BGP antigen | Partially purified BGP |
|---|---|---|
| | Moles/100 moles | |
| Aspartic acid | 7.8 | 7.2 |
| Threonine | 8.3 | 8.0 |
| Serine | 10.0 | 7.2 |
| Glutamic acid | 16.9 | 16.5 |
| Proline | 12.4 | 12.9 |
| Glycine | 7.8 | 8.9 |
| Alanine | 4.3 | 5.6 |
| Valine | 5.3 | 5.4 |
| Methionine | 0.3 | 1.2 |
| Isoleucine | 5.2 | 6.4 |
| Leucine | 9.5 | 9.2 |
| Tyrosine | 0.8 | 0.9 |
| Phenylalanine | 3.0 | 2.8 |
| Histidine | 0.5 | 1.3 |
| Lysine | 2.9 | 1.8 |
| Arginine | 5.3 | 4.8 |
| Total | 100.3 | 99.9 |

# 0 061 912

It will be understood that the homogeneous BGP of this invention has essentially the same amino acid composition as the partially purified BGP, allowing for individual variations in samples from different animals. The two forms differ principally in the substantially complete removal of complex glycolipid from the latter to produce the former. Thus, the homogeneous BGP as determined on a sample from a different bovine individual from that donating the Table III sample, has been found to have an amino acid composition given in Table IV below.

Table IV gives the amino acid compositions of each homogeneous glycoprotein of this invention in terms of moles/100 moles of amino acids present.

TABLE IV

Amino acid composition of horse erythrocyte glycoprotein and of sheep erythrocyte glycoprotein

| Amino acid | Cow, moles/100 moles | Horse, moles/100 moles | Sheep, moles/100 moles |
|---|---|---|---|
| 1/2 Cystine | 0.5 | ND | ND |
| Aspartic acid | 6.2 | 8.1 | 5.6 |
| Threonine[a] | 8.8 | 10.6 | 8.1 |
| Serine[b] | 7.5 | 10.8 | 12.9 |
| Glutamic acid | 16.4 | 9.4 | 13.0 |
| Proline | 13.9 | 12.3 | 11.6 |
| Glycine | 9.9 | 9.2 | 7.7 |
| Alanine | 5.0 | 11.3 | 9.6 |
| Valine | 5.0 | 4.4 | 6.2 |
| Methionine | 1.8 | 0.8 | 0.5 |
| Isoleucine | 5.3 | 3.5 | 4.6 |
| Leucine | 7.7 | 8.2 | 8.3 |
| Tyrosine | 0.5 | 1.1 | 1.6 |
| Phenylalanine | 3.0 | 2.9 | 1.2 |
| Histidine | 1.1 | 1.2 | 1.6 |
| Lysine | 2.6 | 1.3 | 3.2 |
| Arginine | 4.8 | 4.8 | 4.0 |
| Tryptophan | 0.2 | ND | 0.3 |

[a] corrected for 5% loss on hydrolysis.
[b] corrected for 10% loss on hydrolysis.
ND=not done.

Individual amino acids were determined after hydrolysis in 6 NHCl at 110°C in sealed, evacuated tubes for 24 hours with a JEOL 5AH amino acid analyzer. N-terminal amino acid analysis was performed by the dansyl method [Gray, W. R. *Methods Enzymol. 25,* 333 (1972)]. DNS-amino acids were identified by TLC as described by Woods, K. R. et al *Biochem. Biophys. Acta. 133,* 369 (1967). For amino acid analysis of the cow and sheep glycoprotein, tryptophan was determined after hydrolysis for 20 hours at 100°C in 4N methane-sulfonic acid.

Cysteine values for BGP was determined by alkylating homogeneous BGP with iodoacetate prior to hydrolysis. The observed differences in amino acid composition between partially purified BGP (Table III)

9

and homogeneous BGP (Table IV) are insubstantial and are attributed, *inter alia*, to the dependence of extent of HCl hydrolysis on the presence of contaminating macroglycolipids, to different amino acid analysers used to analyse each form of BGP, and to well-recognized individual differences among cows used to prepare each form of BGP. It will be understood that the amino acid composition of the partially purified BGP is substantially the same as that of the homogeneous BGP.

A special embodiment of the present invention requires that a tag be attached either to the antigen to be determined or its binding partner. A radioactive tag is preferred, for example, [125]I [131]I, $^3$H or $^{14}$C, or any other radioisotope easily and conveniently measured. Tritium labelling of proteins, for example, can be carried out with [$^3$H]-acetic anhydride, a well known reagent frequently used for this purpose. The isotope [125]I is the most preferred because of its high specific activity and because of its relatively long half life.

An antibody or proteinaceous antigen can be labelled with [125]I to a specific activity of about 50 micro Ci/microgram of protein by one of a variety of methods known in the art such as e.g. lactoperoxidase, chloramine-T [Hunter, W. M. et al *Nature 194*, 405 (1962)], Iodogen [Fraker, P. J. et al *Biochem, Biophys. Res. Comm. 80*, 849 (1978)], or Bolton-Hunter reagent [Bolton, A. E. et al *Biochem. J. 133*, 529 (1973)]. The labelled protein is separated from unreacted free [125]I by gel filtration.

The preferred method of radioactively labelling proteins containing tyrosine is chloramine-T. It is the most frequently used method of labelling proteins with a radioactive tag. Alternatively, one can label tyrosine residues with tritium by the two-step process of first reacting protein with NaI, then catalytically dehydrogenating with tritium gas. The free amino group of the side chains of lysine or of arginine can be labelled with $^3$H-acetic anhydride or with Bolton-Hunter reagent.

A large variety of other tags can be employed instead of radioactive ones. Other methods of tagging include attachment of a fluorogen, chromophore, enzyme or luminescent tag. For example, horse radish peroxidase or beta-galactosidase may be coupled to a proteinaceous antigen by many of the methods discussed in Kennedy, J. H. et al, *Clinica Chimica Acta 70*, 1 (1976). Fluorescein isothiocyanate is a well known fluorogenic group that can be attached to proteins [Ackroyd, J. F. ed., *Immunological Methods*, Blackwells Oxford (1964) pp. 155—174].

A. Preparation of a crude antigen from bovine, horse or sheep red blood cells

Hemoglobin-free stroma is isolated by extraction of stroma, i.e. membranes, as generally described in Fletcher et al., *J. Imm. 107*, 842 (1971). More particularly, to fresh blood from newly slaughtered cattle, an anticoagulant is added to prevent clotting. Almost any conventional anticoagulant may be used, except that heparin and ethylenediamine tetraacetic acid must be avoided. Preferred anticoagulants include acidified citrate-dextrose, oxalate and Alsievers. The blood may be stored at normal refrigeration temperatures for up to about 24 hours. It is then thoroughly washed with any of the common isotonic buffers at a pH that preserves the integrity of the cell membrane, i.e., in the order of about 5.0 to about 9.0, preferably about 6.0 to about 8.0 such as phosphate-buffered saline of pH about 7.4, or Tris-buffered saline of pH about 7.4. If the blood samples are allowed to stand for a period appreciably longer than about 24 hours prior to washing, the samples may become contaminated with bacteria or with lysosomal enzymes from white blood cells. For each washing step, the cells are suspended in buffer and then centrifuged. After centrifugation, the supernatant and buffy coat are removed by aspiration. These washing-centrifugation-aspiration steps are continued until the buffy coat is substantially completely removed and the supernatant is essentially clear and free from color. It is important to remove substantially all of the buffy coat because it is a source of hydrolytic enzymes. Approximately one quarter of the total volume of erythrocytes is typically lost during the washing steps.

Washed and packed erythrocytes are then lysed in hypotonic buffer of alkaline pH, i.e. above at about 8.0. The lysing buffer must be alkaline to prevent denaturation of hemoglobin and unwanted binding of denatured hemoglobin to membranes. Cell stroma or membranes are packed together by centrifugation at about 13,000×g for 1 hour at a temperature in the order of about 4° to about 15°C. The supernatant is aspirated and discarded, whereupon the loosely packed membrane layer is decanted from the red pellet. This procedure of alkaline washing followed by cold centrifugation and separation steps is repeated until the decanted membrane layer is creamy white, i.e., without visible hemoglobin contamination. The purified stroma must then be dried, e.g., by freeze-drying to carry out the next steps.

An alternative method to centrifugation is filtration on Pellicon (Trademark of Millipore), as described in Rosenberry, T. L. et al *J. Biochem. Biophys. Meth. 4*, 39 (1981) for the preparation of membranes from human erythrocytes.

Thereafter, an even suspension in acetone of freeze-dried, hemoglobin-free stroma from cow, horse or sheep erythrocytes is prepared by grinding in a mortar and pestle about 1 gram of stroma with, preferably about 200 ml acetone. The acetone must be anhydrous, and taken from a freshly opened bottle. The volume of acetone per gram of stroma may vary from as low as about 100 ml up to a volume substantially greater than 200 ml. The suspension is refluxed, normally for about 3 hours, but the time may vary from about 1 to 6 hours. The residue is collected by filtering off the acetone while the suspension is still warm; otherwise there is some unwanted precipitation of acetone-soluble neutral lipids on the filter. The residue is thoroughly washed with acetone preferably warm acetone, and air dried.

The same refluxing procedure is repeated on the acetone extracted residue, this time substituting anhydrous, 100% ethanol for the acetone. Extraction with 100% ethanol removes some but not all of the glycolipids. The ethanol extract is discarded, and the residue is dried. The acetone and ethanol extract residue is refluxed again, preferably with about 75% aqueous ethanol but a solution of between about 50% and about 80% aqueous ethanol may be used. The supernatant is removed by filtration and set aside. The residue is then washed with an equal volume of the aqueous ethanol. The washed residue is discarded. The extract and all the washings are combined, concentrated, dialyzed to remove salt and freeze-dried in a conventional manner. Alternatively, the aqueous ethanol extract and its washings may be combined, concentrated, dialyzed against water and stored under normal refrigeration temperatures.

B. Purification of bovine, horse or sheep glycoproteins to homogeneity

The lyophilized aqueous ethanol extract from Step A is redissolved in water and precipitated with about 90% aqueous ethanol. Incubation on ice for at least 2 hours and addition of a salt, e.g., 3 or 4 crystals of sodium acetate or other common salt, can help to initiate precipitation. Typically, a massive, white precipitate appears. This precipitate is centrifuged and the supernatant is removed and discarded. The precipitate is then dialyzed against a conventional buffer of low ionic strength and low pH in preparation for passage through a cationic exchange column.

Chromatography on a cationic exchange resin is best performed at a pH of from about 4 to about 6, e.g., above the isoelectric focusing point of the glycoprotein reactive with heterophile antibodies. Resins appropriate for this step include phosphocellulose, carboxymethyl cellulose, and any of the weakly acidic acrylic resins commonly employed for purification of proteins. After the resin is pre-equilibrated with the low ionic strength, low pH buffer, the dialyzed protein sample is passed through the column. Neutral and basic contaminants are bound by the column and discarded. Those fractions which contain sialic acid and do not bind the column are collected. They can be dialyzed against water and freeze-dried or lyophilized if desired.

The collected fractions, preferably but not necessarily in lyophilized form, are next treated. If lyophilized or otherwise dried, the fractions are first dissolved in water, about 5 volumes of diethyl ether:ethanol (in a volume to volume ratio ranging from about 4:1 to about 1:1) are next added and the mixture is centrifuged and permitted to settle. Two phases appear, one aqueous and one comprising diethyl ether. The aqueous layer is removed and freeze-dried to remove residual ether and water.

Another extraction with a different lipid solvent from diethyl ether is then performed, in accordance with conventional techniques of chemistry. In this procedure, the lyophilized extract from the first extraction step is dissolved in water and re-extracted in about 9 volumes of chloroform/methanol in the ratio of about 2:1, volume by volume. The mixture is again centrifuged and permitted to separate into an aqueous phase and an organic phase. The separated aqueous phase is removed and lyophilized. If the lyophilized material originates from cow, it is the partially purified BGP of this invention.

In order to remove contaminants still remaining principally comprising complex glycolipids, the lyophilized glycoprotein is then dissolved in a low ionic strength buffer containing about 1% neutral detergent, such as Emulphogen or Triton-X-100. This detergent aids in dissolving the complex glycolipid. The solution is loaded onto an anion exchange column, pre-equilibrated with low ionic strength buffer. A variety of suitable anion exchange resins are commercially available, including DE-52 cellulose, DEAE B10-GEL A and Cellex D. The column is washed with the low ionic strength aqueous buffer until the optical density of the fractions eluted has returned to the level of a blank solution consisting of the buffer alone, normally less than 0.1 units at 280 nanometers. The column is then eluted with aqueous buffer containing high salt concentration, i.e. in the order of more than 0.3 molar concentration of a common salt such as NaCl, Na acetate, KCl, etc. High concentrations of salt compete with the electrostatic interactions between the glycoprotein and the cationic resin, so as to pool a single peak of material. The material is dialyzed against $H_2O$, freeze-dried and held for use.

Polyacrylamide gel electrophoresis in a detergent such as SDS, of the dialyzed, freeze-dried material previously chromatographed on an anion exchanged column yields essentially one band after staining with either a Coomassie blue dye or with a periodic acid Schiff reagent. Polyacrylamide gel electrophoresis in detergents is a well known technique for ascertaining homogeneity of a protein sample and for estimating molecular weight. See, for example, Fletcher, M. A., et al *Biochem. Biophys. Acta. 278*, 163 (1972).

C. Use of the purified glycoproteins of the present invention

In experiments conducted by the applicant, the glycoprotein products of this invention were strong inhibitors of the agglutination of sheep erythrocytes by sera from patients with infectious mononucleosis. The receptor properties were abolished by prior treatment of the glycoproteins with neuraminidase.

It will be understood that any of the immunoassays described herein, or any apparent modifications thereof, are applicable to the purified forms of the glycoproteins of this invention, which forms include the essentially homogeneous BGP, the essentially homogeneous HGP, and the essentially homogeneous SGP.

Any of the glycoprotein products of this invention is useable in the latex bead test for diagnosing and/or monitoring human infectious mononucleosis.

1. Latex bead test

Chemical coupling of any of the purified glycoproteins of this invention to a carrier particle can be accomplished in a variety of ways. Particles composed of a synthetic polymer such as polystyrene, poly(methyl methacrylate) or any synthetic latex are preferred over microbial cells due to their enhanced stability during long term storage. In general, the beads or particles should have free functional groups such as one or more of the primary amino, sulfhydryl, carboxyl or hydroxyl groups. Various methods of coupling proteins to carrier particles composed of a synthetic polymer are discussed in the literature, including *inter alia* in U.S. Patents 4,046,723; 3,882,224; 3,857,931; 3,639,558. In the preferred method of coupling latex beads to a glycoprotein of this invention, the glycoprotein is added to a suspension of about 0.25% (in water) of carboxyl-modified, uniform latex particles. The mixture is stirred for at least about 30 minutes. The pH is maintained between about 4 to and about 6. Then a substituted carbodiimide such as, e.g., 1 - ethyl - 3 - (3 - dimethyl amino propyl) carbodiimide-hydrochloride, is added, in a freshly made solution with a minimum of water. This carbodiimide is added in an amount to provide a final concentration thereof in the order of about 0.1 M. A pH below 7 is maintained for at least one hour, and the mixture is then stirred overnight or longer. The particles are washed alternatively in an aqueous buffer of low pH (about 4.0) then in an aqueous buffer of higher pH (about 8), and finally washed twice with distilled water. The beads containing coupled glycoprotein of this invention are then stored in the higher aqueous pH buffer (about 8) at concentrations of about 1%.

To conduct a test of infectious mononucleosis, glycoprotein-latex beads are diluted in buffer to a concentration in the order of about 0.25% (w/v). Appreciably lower concentrations of the reagent prevent agglutination that is easily visible, while appreciably higher concentrations result in waste of protein bound carrier particles. Serial dilutions in buffer are made of the test antiserum or biological fluid such as serum. One drop of each such dilution is added to one drop of glycoprotein-latex bead reagent within the ceramic ring of a well in a serological ring slide. The slide is rotated briefly to mix antiserum or serum and latex reagent, and agglutination is determined by visual inspection. The end points of the titration are determined as the highest dilution (i.e., most dilute concentration) of serum giving visible clumping. Other containers may be used for the agglutination test, such as the polystyrene microtiter plates with U-shaped wells from Cooke Engineering Co., but agglutination is more difficult to read in such wells.

### 2. Immunoassay using homogeneous glycoproteins of this invention

Many types of immunoassays, including radioimmunoassays, are known in the art and can be employed for the detection and/or quantitation of homogeneous glycoprotein of this invention. An immunoassay may use, for example, a radioactive tag for the antigen, such as $^{125}I$, $^{131}I$, $^{3}H$ or $^{14}C$, or any other radioisotope easily and conveniently measured. Other methods of tagging the antigen include attachment of a fluorogen, chromophore, enzyme or luminescent tag. Numerous methods of separating antibody-antigen complexes from free, (i.e. unbound) antigen are known, including precipitation of antibody (double antibody). Among other known techniques are those using solid support matrices, also known as a solid-phase assay wherein glass, silica or plastic beads or unitary plastic inserts are used to fix one component of the antigen-antibody pair.

A preferred way to tag homogeneous glycoprotein of this invention is with $^{125}I$, a radioactive label that can yield labelled antigens with very high specific activity, as measured in terms of the rate of detectable disintegrations per microgram of protein.

The glycoprotein is labelled with $^{125}I$ to a specific activity of about 1 micro Curie/microgram of protein by one of a variety of methods known in the art such as e.g., lactoperoxidose, chloramine-T labelling, or Iodogen [Fraker, P. J. & J. C. Speck, *Biochem. Biophys. Res. Commun. 80.* 849 (1978)]. The labelled protein is separated from unreacted free $^{125}I$ by gel filtration.

A preferred immunoassay based on $^{125}I$-labelled homogeneous glycoprotein, e.g. $^{125}I$-BGP, is the competitive radioimmunoassay. The principle of the competition radioimmunoassay is that labelled antigens compete with unlabelled antigens for the same combining sites of the antibody in the serum. See, e.g., Kabat, E. (1976) *Structural Concepts in Immunology* and *Immunochemistry* Holt, Richard and Winston. Separation of antibody-antigen complexes from free antigen allows the determination of the amount of antigen bound. This well-known assay has many variations, for example, testing a variety of unlabelled antigens to determine which unlabelled antigen has the greatest similarity to the labelled antigen. Another variation is to test a standardized quantity of radio-labelled and unlabelled antigen against samples from each of many dilutions of a variety of test antisera with the purpose of obtaining a quantitative measure of the titer of specific antibody in any antiserum. This is done by determining the relative amount of radioactive antigen bound by the test antiserum and comparing this quantity with the amount bound by a control antiserum of the same dilution as the test antiserum.

It should be noted that variations within the scope of this invention also include competitive radioimmunoassays using non-radioactive tags for the glycoprotein, such as a fluorogen, chromophore, luminescent material or enzyme.

There are many methods known in the art for separating antibody-antigen complexes from free antigen. One method usable with e.g. homogeneous BGP of this invention is the solid-phase, sandwich-type immunoassay. In this method, microtiter plates, preferably made of a material easily cuttable by scissors and with round bottom wells, are coated by incubation in each well of about two micrograms homogeneous BGP with about 200 microliters of aqueous buffered saline solution overnight. Most suitably, this step is effected in a moist chamber to prevent drying.

12

The wells are then emptied by aspiration, washed at least once with aqueous buffered saline and filled with a solution of protein carrier in aqueous buffered saline. The protein carrier blocks any binding sites on the inside of the well, or the solid-phase, that were not bound by purified BGP added in the first step. Typical solutions of protein carrier used for coating wells are e.g., gelatin, goat serum albumin, rabbit serum albumin, or egg ovalbumin in the concentration ranges of from about 0.05% to about 0.2%. In this assay, *bovine* serum albumin should not be used as a protein carrier because it might interfere with specific binding to homogeneous *bovine* glycoprotein. The plates are incubated with carrier solution for at least 1 hour with gentle agitation or mixing to enhance uniform coating of the well.

The wells are then emptied again by aspiration, washed two to five times with aqueous buffered saline, preferably three times, and then about 200 microliters of suitably diluted serum sample is added to each well. This step binds the antigen to specific antibody. The plates are incubated to equilibrate the antibody with the antigen, for instance at room temperature overnight, preferably in a moist chamber to prevent drying.

The wells are emptied a third time by aspiration, washed about 5 times, then about 10,000 cpm (counts per minute) of $^{125}$I-homogeneous BGP suitably diluted in carrier protein (e.g., 0.1% egg ovalbumin in aqueous buffered saline) is added to each well in a total volume of about 200 microliters. Radiolabelled protein is allowed to equilibrate with binding sites on the antibody still available to the antigen, with, for example, an incubation overnight.

The contents of each well are aspirated for the fourth time, and each well washed at least three times with aqueous buffered saline. The plates are air dried about 6 hours, then their wells are cut out with scissors, and radioactivity is counted in a conventional counter. For $^{125}$I, one minute counts are normally sufficient for reasonable accuracy.

The following examples further illustrate the products of this invention. It will be understood that the glycoproteins of the following examples may be any of BGP, HGP or SGP.

Example 1
Preparation of hemoglobin-free stroma from blood

Fresh blood was collected from sheep at the time of slaughter and from horse by venipuncture in an anti-coagulant citrate-dextrose solution. The blood was held at 4°C for about 24 hours and was centrifuged at about 2500 RPM for about twenty minutes at 4°C to pellet erythrocytes. The supernatant and intermediate cell layer were removed. The erythrocytes were washed three times in an equal volume of cold 0.12 M NaCl, 0.05 sodium phosphate buffer, about pH 7.4.

For each washing step, the cells were resuspended in buffer, then centrifuged at about 2500 RPM for about twenty minutes. After centrifugation, the supernatant and remaining buffy coat were aspirated. This procedure was repeated three times resulting in a 25% loss in the total volume of erythrocytes in order to remove the buffy coat.

Example 2
Isolation and purification of homogeneous glycoprotein

Washed erythrocytes were lysed in nine volumes of cold 0.005 M sodium phosphate at a pH of about 8.0. The membranes were pelleted by centrifugation at 13,000×g for 45 minutes at 4°C. The supernatant was aspirated and discarded. The cell membrane layer was then decanted from the red pellet. The procedure described above was repeated until the membranes were a creamy white color. The membranes were sequentially extracted under reflux with acetone, absolute alcohol and with 75% ethanol. The 75% ethanol extract was concentrated to a small volume by vacuum distillation, dialyzed and freeze-dried. This was the crude extract which was further purified in a series of steps. The 75% ethanol extract was precipitated from ethanol, then dissolved in 0.02 M sodium citrate, pH 4.1 and placed on a phosphocellulose column equilibrated and eluted with the same buffer. The column effluent was monitored for sialic acid, hexose and optical density at 280 nanometers. The sialic acid fraction emerged in the void volume and was dialyzed. Glycoprotein, at a concentration in water of 2 mg/ml was mixed for thirty minutes at 0°C with 5 volumes of diethyl ether:ethanol (4:1 volume/volume). The aqueous phase was lyophilized and extracted again with diethyl ether:ethanol. Glycoprotein, at a concentration of 10 mg/ml water was extracted with nine volumes of chloroform:methanol (2:1 volume/volume) for 30 minutes at 23°C and was centrifuged at 2000 RPM for 30 minutes. The aqueous layer was lyophilized and dissolved in 0.05 M Tris-HCl buffer at pH of 8.0 containing 1% Emulphogene BC-720 (GAF, New York N.Y.) and placed on a Whatman DE-52 cellulose column equilibrated with 0.5 M Tris-HCl buffer at pH 8.0. This column was eluted with a 0.0→0.5 M NaCl gradient buffer. The purified glycoprotein eluted as a single peak and was dialyzed and freeze-dried.

Example 3
Erythrocyte glycoprotein-latex bead reagent

The purified erythrocyte glycoprotein isolated as in Example 2 above, was coupled to a 0.25% suspension in water of carboxyl-modified uniform latex particles, 0.455 micron average diameter (Dow Diagnostics). The mixture was stirred with the pH maintained as 5±1 for 30 minutes at 22 to 25°C. Then 1-ethyl-3-(3-dimethylaminopropyl) carbodiimide-hydrochloride, freshly dissolved in a minimum amount of water, was added to a final concentration of 0.1 M. The pH was maintained at 5.5 for 1 hour, and the mixture was stirred at 22 to 25°C for 18 hours. The particles were then washed alternatively in a buffer containing 0.1 M glycine, 0.15 M NaCl, 7 mM $CaCl_2$ (GBS) at pH 4.0 and then at pH 8.0 and twice with distilled water. Between each wash the particles were centrifuged at 17,000×g for 20 minutes. The glycoprotein latex bead reagent was then diluted to a concentration of 1% in GBS at pH 8.3 and stored at 4°C. Before use, the reagent was diluted to 0.25% with GBS.

Example 4
Latex slide test with glycoprotein latex coupled beads
The glycoprotein-latex bead reagent of Example 3 was used in a latex test carried out on serological ring slides, 7.9 by 12.1 cm (Scientific Products). Serial doubling dilutions of test sera were done in GBS (pH 8.3). One drop of each dilution of serum was then added to one drop of the glycoprotein latex bead reagent within a ceramic ring on the slide. The slide was rotated on a Thomas Clinical Rotater at 1,500 RPM for 5 minutes at 22 to 25°C and agglutination was determined by microscopic examination. The endpoints of titration were recognized as the highest dilution of serum giving visible clumping as compared to a control latex suspension.

Example 5
Purified glycoprotein labelled with [125]I
One hundred micrograms of the purified glycoprotein from Example 2 is labelled with 100 microcuries of [215]I. A 20 microliter aliquot of methylene chloride solution containing 0.4 micrograms 1,3,4,6-tetra-chloro-3a,6a-diphenyl glycoluril (IIa) (Pierce Chemical Co. Rockford, III.) is placed in a test tube, and the methylene chloride is evaporated by rotating the tube in a 37°C bath so that a thin film of IIa forms in the bottom. To another test tube is added, in pH 8.2 borate-saline buffer, 100 micrograms of the purified glycoprotein, 0.11 micrograms of potassium iodide and 100 microcuries of [125]I; the final volume of this mixture is adjusted to 100 μl with borate-saline buffer. After bringing the tubes to 0—2°C, reaction is initiated by transferring the glycoprotein solution to the tube containing IIa. The reaction is allowed to proceed for 5 minutes at 0—2°C with gentle stirring; it is terminated by decanting the mixture from the residual glycouril. For determining the efficiency of iodination, 5 microliter aliquots of the reaction mixture are mixed with 0.1 mg of bovine serum albumin, in 10 microliters of borate-saline, and then with 1 milliliter of cold 10% trichloroacetic acid. After standing for 30 minutes at 0—2°, these mixtures are centrifuged. The pellets are mixed with 1 milliliter of fresh 10% trichloroacetic acid, and the mixtures are centrifuged again. The protein precipitates are then dissolved in 1 milliliter of 0.1 M sodium hydroxide. The labelled protein is separated from free iodine by chromatography on Sephadex G-50 column. The specific activity is 1 microCurie per microgram glycoprotein.

Example 6
Glycoprotein radioimmunoassay
This solid-phase, sandwich type immunoassay uses the homogeneous glycoprotein of Example 2. The wells of polystyrene microtitre plates (U-bottom, Cooke Engineering Co., U.S.A.) were coated with purified glycoprotein by incubating with 2 micrograms of the purified glycoprotein in 200 microliters of phosphate-buffered saline (PBS). The plates incubated overnight at 22—25°C in a moist chamber. The wells were emptied by aspiration and washed three times in PBS. Then the wells were filled with 0.1% gelatin in PBS and incubated at 22—25°C for 1 hour on a Cortis Laboratories micromixer. The wells were then emptied by aspiration, washed three times in PBS and 200 microliters of suitably diluted serum was added to each well. The plates were incubated overnight at 22—25°C in a moist chamber. The wells were emptied and washed in two liters of PBS with mixing. The [125]I-labelled erythrocyte glycoprotein suitably diluted in 0.05% bovine serum albumin (BSA) in PBS was added to each well in a volume of 200 microliters. The plates were incubated overnight. The contents of each well were aspirated and washed three times with PBS. The plates were air dried. Each well was cut out with scissors and radioactivity counted in a gamma scintillation counter.

Example 7
E-Rosette inhibition assay
Human lymphocytes were isolated from samples of peripheral heparinized venous blood. The lymphocytes were isolated by gradient centrifugation. The lymphocyte isolation was carried out by layering the blood, which had been diluted 1:2 with RPMI medium, above lymphocyte separation medium (LSM, Litton Bionetics). The tubes were centrifuged at about 400×g for about 45 minutes. Mononuclear cells were collected from the interphase, washed and counted. Various amounts of glycoprotein were incubated with the peripheral blood lymphocytes at about 20°C for about 30 minutes. Washed sheep erythrocytes were then added to effect a ratio of erythrocytes to peripheral blood lymphocytes of 40:1. The mixture was centrifuged at about 200×g for about 5 minutes at about 20°C then incubated at about 40°C overnight before rosettes were counted. Three or more erythrocytes attached to a lymphocyte were considered a rosette.

14

## 0 061 912

**Claims**

1. A process for preparing a bovine, horse or sheep erythrocyte glycoprotein, which comprises the steps of:

(a) uniformly suspending dried, ground, hemoglobin-free stroma from the appropriate erythrocytes in anhydrous acetone;

(b) refluxing for from about 1 to about 6 hours, filtering and drying the residue;

(c) suspending said dried residue in 100% anhydrous ethanol;

(d) refluxing for from 1 to about 6 hours, filtering and drying the residue;

(e) suspending the dried residue from step (d) in aqueous ethanol of from about 50% to about 80% strength and repeating step (b);

(f) dissolving the residue from step (e) in water and adding 90% aqueous ethanol, followed by incubating on ice, until crystallization occurs, centrifuging and dialyzing the solid layer against a low pH, low ionic strength buffer;

(g) passing the solid from step (f) through a cation exchange resin on a chromatographic column;

(h) collecting the sialic acid containing fractions from the column and drying them;

(i) treating the collected fractions from step (h) by extraction with a known lipid solvent, centrifuging, collecting the aqueous layer and drying it;

(j) repeating step (i) on the product of that step, using a different lipid solvent; and

(k) recovering the product of step (j) in lyophilised form; characterised in that complex glycolipid is removed from the product of step (k) by:

(l) dissolving the product of step (k) in a low ionic strength buffer containing about 1% neutral detergent;

(m) loading the solution from step (l) on an anion exchange chromatographic column;

(n) washing the column thoroughly with low ionic strength buffer;

(o) eluting the column with aqueous buffer to high salt concentration; and

(p) dialyzing the product of step (o) against water and recovering the product in freeze dried form.

2. A process according to claim 1 wherein the cation exchange resin is phosphocellulose.

3. A process according to claim 1 or claim 2 wherein the lipid solvent of step (i) is diethyl ether and ethanol in a ratio by volume between about 4:1 and about 1:1.

4. A process according to any one of claims 1 to 3 in which the lipid solvent of step (j) is chloroform and methanol in a volume ratio of about 2:1.

5. A process according to any one of claims 1 to 4 wherein the solvent in step (e) is 75% aqueous ethanol.

6. An essentially homogeneous bovine erythrocyte glycoprotein obtainable by the process of claim 1, the amino acid composition of which is in the order of about 7.2 mole % aspartic acid, about 8 mole % threonine, about 7.2 mole % serine, about 16.5 mole % glutamic acid, about 12.9 mole % proline, about 8.9 mole % glycine, about 5.6 mole % alanine, about 5.4 mole % valine, about 1.2 mole % methionine, about 6.4 mole % isoleucine, about 9.2 mole % leucine, about 0.9 mole % tyrosine, about 2.8 mole % phenylalanine, about 1.3 mole % histidine, about 1.8 mole % lysine and about 4.8 mole % arginine.

7. The bovine glycoprotein of claim 6, having a carbohydrate content of approximately 25 weight per cent, comprising hexose, sialic acid, N-acetylgalactosamine and N-acetylglucosamine in the approximate molar ratio of 1.6:1.0:0.5:1.1.

8. An essentially homogeneous horse erythrocyte glycoprotein obtainable by the process of claim 1, the amino acid composition of which is about 8.1 mole % aspartic acid, about 10.6 mole % threonine, about 10.8 mole % serine, about 9.4 mole % glutamic acid, about 12.3 mole % proline, about 9.2 mole % glycine, about 11.3 mole % alanine, about 4.4 mole % valine, about 0.8 mole % methionine, about 3.5 mole % isoleucine, about 8.2 mole % leucine, about 1.1 mole % tyrosine, about 2.9 mole % phenylalanine, about 1.2 mole % histidine, about 1.3 mole % lysine and about 4.8 mole % arginine.

9. The horse glycoprotein of claim 8, which is a sialoglyprotein comprised of about 55% carbohydrate residues in a composition of about 28 grams N-glycolneuraminic acid, about 11 grams galactose, about 14 grams N-acetylgalactosamine, about 1 gram N-acetylglucosamine and about 1 gram mannose per 100 grams of glycoprotein.

10. An essentially homogeneous sheep erythrocyte glycoprotein obtainable by the process of claim 1, the amino acid composition of which is about 5.6 mole % aspartic acid, about 8.1 mole % threonine, about 12.9 mole % serine, about 13.0 mole % glutamic acid, about 11.6 mole % proline, about 7.7 mole % glycine, about 9.6 mole % alanine, about 6.2 mole % valine, about 0.5 mole % methionine, about 4.6 mole % isoleucine, about 8.3 mole % leucine, about 1.6 mole % tyrosine, about 1.2 mole % phenylalanine, about 1.6 mole % histidine, about 3.2 mole % lysine, about 4.0 mole % arginine, and about 0.3 mole % tryptophan.

11. The sheep glycoprotein of claim 10, which is a sialoglycoprotein comprised of about 56.6% carbohydrate residues in a composition of about 16.7 grams sialic acids, about 9 grams galactose, about 13.7 grams N-acetylgalactosamine, about 14.4 grams N-acetylglucosamine, and about 1.7 grams mannose per 100 grams of glycoprotein.

12. A process for diagnosing infectious mononucleosis which comprises the steps of:

15

(a) appending to minute particles of latex or synthetic resin a glycoprotein of any one of claims 6 to 11 or made by a process of any one of claims 1 to 5;

(b) coating a flat surface with particles from step (a) hereof;

(c) adding a diluted sample of biological test fluid from a patient suspected of harboring infectious mononucleosis and rotating the flat surface;

(d) observing the degree of agglutination of the sample; and

(e) comparing the result of step (d) with the degree of agglutination observed when a standard containing a known amount of infectious mononucleosis heterophile antibody is substituted as the sample in step (c) of the procedure recited in steps (a) through (d).

13. A glycoprotein according to any one of claims 6 to 11 or made by a process according to any one of claims 1 to 5, containing a label selected from the group consisting of radioisotopes, fluorogens, chromophores, enzymes, luminescent substances and other known labels used in immunoassay procedures.

14. The labelled glycoprotein of claim 13 in which the label is $^{125}$I.

15. A radioimmunoassay procedure for determining the quantity of heterophile antibody of human infectious mononucleosis that is present in a test fluid sample wherein the antigen for said antibody comprises a radio-labelled glycoprotein of claim 13 or claim 14.

16. A procedure according to claim 15 in which the radioimmunoassay is of the sandwich type.

17. A procedure according to claim 15 in which the radioimmunoassay is a competition assay.

18. A radioimmunoassay procedure according to claim 17 in which the glycoprotein is bound to a solid surface.

19. A horse or sheep erythrocyte glycoprotein according to any one of claims 7 to 11 or made by a process according to any one of claims 1 to 5 for use as a stable, standardisable reagent for the enumeration of rosetting lymphocytes.

20. A glycoprotein according to any one of claims 6 to 11 or made by a process according to any one of claims 1 to 5 for use as a diagnostic reagent useful in the detection of infectious mononucleosis.

21. A process for the inhibition of rosetting by human blood lymphocytes which comprises incubating said lymphocytes with a horse or sheep glycoprotein according to any one of claims 7 to 11 or made by a process according to any one of claims 1 to 5.

22. A procedure for monitoring or diagnosing human infectious mononucleosis which comprises utilizing a glycoprotein according to any one of claims 6 to 11 or made by a process according to any one of claims 1 to 5 as an inhibitor of agglutination.

**Patentansprüche**

1. Verfahren zur Herstellung eines Rinder-, Pferde- oder Schaf-Erythrozyten-Glykoproteins, umfassend die Stufen:

(a) gleichmässige Suspendierung von getrocknetem, gemahlenem, hämoglobinfreiem Stroma der entsprechenden Erythrozyten in wasserfreiem Aceton;

(b) Rückflussbehandlung etwa 1 bis etwa 6 Stunden, Filtrieren und Trocknen des Rückstandes;

(c) Suspendieren des getrockneten Rückstandes in 100-prozentigem wasserfreien Ethanol;

(d) Rückflussbehandlung während 1 bis etwa 6 Stunden, Filtrieren und Trocknen des Rückstandes;

(e) Suspendieren des getrockneten Rückstandes aus Stufe (d) in wässrigem Ethanol einer Stärke von etwa 50 bis etwa 80% und Wiederholung von Stufe (b);

(f) Lösen des Rückstandes aus Stufe (e) in Wasser und Zugabe von 90-prozentigem wässrigen Ethanol, anschliessend Inkubieren auf Eis, bis Kristallisation auftritt, Zentrifugieren und Dialyse der festen Schicht gegen einen Puffer mit geringem pH und geringer Ionenstärke;

(g) Hindurchleiten des Feststoffes aus Stufe (f) durch ein kationenaustauscherharz in einer chromatographischen Säule;

(h) Sammeln der Sialsäure enthaltenden Fraktionen aus der Säule und deren Trocknung;

(i) Behandlung der gesammelten Fraktionen aus Stufe (h) durch Extraktion mit einem bekannten Lipid-Lösungsmittel, Zentrifugieren, Sammeln der wässrigen Schicht und deren Trocknung;

(j) Wiederholung von Stufe (i) mit dem Produkt dieser Stufe unter Verwendung eines anderen Lipid-Lösungsmittels; und

(k) Gewinnung des Produktes aus Stufe (j) in lyophilisierter Form; dadurch gekennzeichnet, dass komplexes Glykolipid aus dem Produkt der Stufe (k) entfernt wird durch:

(l) Lösen des Produktes von Stufe (k) in einem Puffer mit geringer Ionenstärke, der etwa 1% neutrales Detergens enthält;

(m) Aufbringen der Lösung aus Stufe (1) auf eine chromatographische Anionenaustauschersäule;

(n) sorgfältiges Waschen der Säule mit einem Puffer mit geringer Ionenstärke;

(o) Eluieren der Säule mit wässrigem Puffer zu hoher Salzkonzentration; und

(p) Dialyse des Produktes aus Stufe (o) gegen Wasser und Gewinnung des Produktes in gefriergetrockneter Form.

2. Verfahren gemäss Anspruch 1, worin das Kationenaustauscherharz Phosphozellulose ist.

3. Verfahren gemäss Anspruch 1 oder Anspruch 2, worin das Lipidlösungsmittel von Stufe (i) Diethylether und Ethanol in einem Volumenverhältnis zwischen etwa 4:1 und etwa 1:1 ist.

4. Verfahren gemäss einem der Ansprüche 1—3, worin das Lipidlösungsmittel von Stufe (j) Chloroform und Methanol in einem Volumenverhältnis von etwa 2:1 ist.

5. Verfahren gemäss einem der Ansprüche 1—4, worin das Lösungsmittel in Stufe (e) 75-prozentiges wässriges Ethanol ist.

6. Im wesentlichen homogenes Rindererythrozytenglykoprotein, erhältlich nach dem Verfahren von Anspruch 1, dessen Aminosäurezusammensetzung grössenordnungsmässig etwa 7,2 Mol-% Asparaginsäure, etwa 8 Mol% Threonin, etwa 7,2 Mol-% Serin, etwa 16,5 Mol-% Glutaminsäure, etwa 12,9 Mol-% Prolin, etwa 8,9 Mol-% Glycin, etwa 5,6 Mol-% Alanin, etwa 5,4 Mol-% Valin, etwa 1,2 Mol-% Methionin, etwa 6,4 Mol-% Isoleucin, etwa 9,2 Mol-% Leucin, etwa 0,9 Mol-% Tyrosin, etwa 2,8 Mol-% Phenylalanin, etwa 1,3 Mol-% Histidin, etwa 1,8 Mol-% Lysin und etwa 4,8 Mol-% Arginin beträgt.

7. Rinderglykoprotein nach Anspruch 6, mit einem Kohlehydratgehalt von etwa 25 Gew.-%, umfassend Hexose, Sialsäure, N-Acetylgalaktosamin und N-Acetylglukosamin in dem ungefähren Mol-Verhältnis von 1,6:1,0:0,5:1,1.

8. Im wesentlichen homogenes Pferdeerythrozytenglykoprotein, erhältlich nach dem Verfahren von Anspruch 1, dessen Aminosäurezusammensetzung etwa 8,1 Mol-% Asparaginsäure, etwa 10,6 Mol-% Threonin, etwa 10,8 Mol-% Serin, etwa 9,4 Mol-% Glutaminsäure, etwa 12,3 Mol-% Prolin, etwa 9,2 Mol-% Glycin, etwa 11,3 Mol-% Alanin, etwa 4,4 Mol-% Valin, etwa 0,8 Mol-% Methionin, etwa 3,5 Mol-% Isoleucin, etwa 8,2 Mol-% Leucin, etwa 1,1 Mol-% Tyrosin, etwa 2,9 Mol-% Phenylalanin, etwa 1,2 Mol-% Histidin, etwa 1,3 Mol-% Lysin und etwa 4,8 Mol-% Arginin beträgt.

9. Pferdeglykoprotein nach Anspruch 8, welches ein Sialoglykoprotein umfassend etwa 55% Kohlehydratreste in einer Zusammensetzung von etwa 28 g N-Glykolneuraminsäure, etwa 11 g Galaktose, etwa 14 g N-Acetylgalaktosamin, etwa 1 g N-Acetylglukosamin und etwa 1 g Mannose pro 100 g Glykoprotein ist.

10. Im wesentlichen homogenes Schaferythrozytenglykoprotein, erhältlich nach dem Verfahren von Anspruch 1, dessen Aminosäurezusammensetzung etwa 5,6 Mol-% Asparaginsäure, etwa 8,1 Mol-% Threonin, etwa 12,9 Mol-% Serin, etwa 13,0 Mol-% Glutaminsäure, etwa 11,6 Mol-% Prolin, etwa 7,7 Mol-% Glycin, etwa 9,6 Mol-% Alanin, etwa 6,2 Mol-% Valin, etwa 0,5 Mol-% Methionin, etwa 4,6 Mol-% Isoleucin, etwa 8,3 Mol-% Leucin, etwa 1,6 Mol-% Tyrosin, etwa 1,2 Mol-% Phenylalanin, etwa 1,6 Mol-% Histidin, etwa 3,2 Mol-% Lysin, etwa 4,0 Mol-% Arginin und etwa 0,3 Mol-% Tryptophan beträgt.

11. Schafglykoprotein nach Anspruch 10, welches ein Sialoglykoprotein umfassend etwa 56,6% Kohlehydratreste in einer Zusammensetzung von etwa 16,7 g Sialsäuren, etwa 9 g Galaktose, etwa 13,7 g N-Acetylgalaktosamin, etwa 14,4 g N-Acetylglukosamin und etwa 1,7 g Mannose pro 100 g Glykoprotein ist.

12. Verfahren zur Diagnose infektiöser Mononukleose, umfassend die Stufen:

(a) Aufbringen eines Glykoproteins nach einem der Ansprüche 6—11 oder hergestellt durch ein Verfahren nach einem der Ansprüche 1—5 auf sehr kleine Teilchen von Latex oder synthetischem Harz;

(b) Beschichten einer flachen Oberfläche mit Teilchen aus Stufe (a) hiervon;

(c) Zugabe einer verdünnten Probe einer biologischen Testflüssigkeit aus einem Patienten, von dem vermutet wird, dass er infektiöse Mononukleose beherbergt, und Rotieren der flachen Oberfläche;

(d) Beobachtung des Agglutinationsgrades der Probe; und

(e) Vergleich des Ergebnisses von Stufe (d) mit dem Agglutinationsgrad, der beobachtet wird, wenn ein Standard mit einem Gehalt einer bekannten Menge von heterophilem Antikörper der infektiösen Mononukleose als Probe in Stufe (c) des in den Stufen (a) bis (d) beschriebenen Verfahrens eingesetzt wird.

13. Glykoprotein gemäss einem der Ansprüche 6—11 oder hergestellt durch ein Verfahren gemäss einem der Ansprüche 1—5, enthaltend eine Markierung ausgewählt aus der Gruppe bestehend aus Radioisotopen, Fluorophoren, Chromophoren, Enzymen, lumineszierenden Substanzen und anderen bekannten Markierungen, die in Immunbestimmungsverfahren eingesetzt werden.

14. Markiertes Glykoprotein nach Anspruch 13, worin die Markierung $^{125}$I ist.

15. Radioimmunbestimmungs verfahren zur Bestimmung der Menge von heterophilem Antikörper der humanen infektiösen Mononukleose, die in einer Testflüssigkeitsprobe vorhanden ist, worin das Antigen für den Antikörper ein radioaktiv markiertes Glykoprotein nach Anspruch 13 oder Anspruch 14 umfasst.

16. Verfahren gemäss Anspruch 15, worin der Radioimmunversuch Schichtstruktur aufweist.

17. Verfahren gemäss Anspruch 15, worin der Radioimmunversuch eine Konkurrenzbestimmung ist.

18. Radioimmunbestimmungsverfahren gemäss Anspruch 17, worin das Glykoprotein an eine feste Oberfläche gebunden ist.

19. Pferde- oder Schaferythrozytenglykoprotein gemäss einem der Ansprüche 7—11 oder hergestellt durch ein Verfahren gemäss einem der Ansprüche 1—5 zur Verwendung als stabiles, standardisierbares Reagens zur Spezifizierung von rosettenbildenden Lymphozyten.

20. Glykoprotein gemäss einem der Ansprüche 6—11 oder hergestellt durch ein Verfahren gemäss einem der Ansprüche 1—5 zur Verwendung als diagnostisches Reagens, das zur Bestimmung der infektiösen Mononukleose brauchbar ist.

21. Verfahren zur Hemmung der Rosettenbildung durch humane Blutlymphozyten, umfassend die

# 0 061 912

Inkubation der Lymphozyten mit einem Pferde- oder Schafglykoprotein gemäss einem der Ansprüche 7—11 oder hergestellt durch ein Verfahren gemäss einem der Ansprüche 1—5.

22. Verfahren zur Überwachung oder Diagnose der humanen infektiösen Mononukleose, umfassend die Verwendung eines Glykoproteins gemäss einem der Ansprüche 6—11 oder hergestellt durch ein Verfahren gemäss einem der Ansprüche 1—5 als Inhibitor der Agglutination.

## Revendications

1. Un procédé pour la préparation d'une glycoprotéine érythrocytaire de bovin, de cheval ou de mouton qui comprend les stades de:

(a) mise en suspension uniforme de stroma dépourvu d'hémoglobine séché et broyé des érythrocytes appropriés dans de l'acétone anhydre;

(b) reflux pendant environ 1 à environ 6 heures, filtration et séchage du résidu;

(c) mise en suspension dudit résidu séché dans de l'éthanol anhydre à 100%;

(d) reflux pendant 1 à environ 6 heures, filtration et séchage du résidu;

(e) mise en suspension du résidu séché du stade (d) dans de l'éthanol aqueux ayant une concentration d'environ 50% à environ 80% et répétition du stade (b);

(f) dissolution du résidu du stade (e) dans de l'eau et addition d'éthanol aqueux à 90% puis incubation sur de la glace jusqu'à ce qu'une cristallisation se produise, centrifugation et dialyse de la couche solide contre un tampon à bas pH et à faible force ionique;

(g) passage du solide du stade (f) à travers une résine échangeuse de cations sur une colonne chromatographique;

(h) recueil des fractions contenant l'acide sialique de la colonne et séchage de celles-ci;

(i) traitement des fractions recueillies du stade (h) par extraction avec un solvant connu des lipides, centrifugation, récolte de la couche aqueuse et séchage de celle-ci;

(j) répétition du stade (i) sur le produit de ce stade en utilisant un solvant différent des lipides; et

(k) récupération du produit du stade (j) sous une forme lyophilisée; caractérisé en ce que le glycolipide complexe est éliminé du produit du stade (k) par:

(l) dissolution du produit du stade (k) dans un tampon de faible force ionique contenant environ 1% de détergent neutre;

(m) chargement de la solution du stade (1) sur une colonne chromatographique d'échange d'anions;

(n) lavage soigneux de la colonne avec un tampon à faible force ionique;

(o) élution de la colonne avec un tampon aqueux ayant une concentration élevée en sel; et

(p) dialyse du produit du stade (o) contre de l'eau et récupération du produit sous une forme lyophilisée.

2. Un procédé selon la revendication 1 dans lequel la résine échangeuse de cations est une phosphocellulose.

3. Un procédé selon la revendication 1 ou la revendication 2 dans lequel le solvant des lipides du stade (i) est constitué d'éther diéthylique et d'éthanol dans un rapport en volume entre environ 4/1 et environ 1/1.

4. Un procédé selon l'une quelconque des revendications 1 à 3 dans lequel le solvant des lipides du stade (j) est constitué de chloroforme et de méthanol dans un rapport en volume d'environ 2/1.

5. Un procédé selon l'une quelconque des revendications 1 à 4 dans lequel le solvant du stade (e) est de l'éthanol aqueux à 75%.

6. Une glycoprotéine érythrocytaire bovine essentiellement homogène pouvant être obtenue selon le procédé de la revendication 1 dont la composition des amino-acides est: de l'ordre d'environ 7,2% molaires d'acide aspartique, environ 8% molaires de thréonine, environ 7,2% molaires de sérine; environ 16,5% molaires d'acide glutamique, environ 12,9% molaires de proline, environ 8,9% molaires de glycine, environ 5,6% molaires d'alanine, environ 5,4% molaires de valine, environ 1,2% molaire de méthionine, environ 6,4% molaires d'isoleucine, environ 9,2% molaires de leucine, environ 0,9% molaire de tyrosine, environ 2,8% molaires de phénylalanine, environ 1,3% molaire d'histidine, environ 1,8% molaire de lysine et environ 4,8% molaires d'arginine.

7. La glycoprotéine bovine de la revendication 6 ayant une teneur en hydrates de carbone d'environ 25% en poids comprenant un hexose, de l'acide sialique, de la N-acétylgalactosamine et de la N-acétylglucosamine dans le rapport molaire approximatif de 1,6/1,0/0,5/1,1.

8. Une glycoprotéine érythrocytaire de cheval essentiellement homogène pouvant être obtenue selon le procédé de la revendication 1 dont la composition des amino-acides est d'environ 8,1% molaires d'acide aspartique, environ 10,6% molaires de thréonine, environ 10,8% molaires de sérine, environ 9,4% molaires d'acide glutamique, environ 12,3% molaires de proline, environ 9,2% molaires de glycine, environ 11,3% molaires d'alanine, environ 4,4% molaires de valine, environ 0,8% molaire de méthionine, environ 3,5% molaires d'isoleucine, environ 8,2% molaires de leucine, environ 1,1% molaire de tyrosine, environ 2,9% molaires de phénylalanine, environ 1,2% molaire d'histidine, environ 1,3% molaire de lysine et environ 4,8% molaires d'arginine.

9. La glycoprotéine de cheval de la revendication 8 qui est une sialoglycoprotéine constituée d'environ 55% de restes d'hydrates de carbone en une composition d'environ 28 g d'acide N-glycolylneuraminique,

18

environ 11 g de galactose, environ 14 g de N-acétylgalactosamine, environ 1 g de N-acétylglucosamine et environ 1 g de mannose pour 100 g de glycoprotéine.

10. Une glycoprotéine érythrocytaire de mouton essentiellement homogène pouvant être obtenue selon le procédé de la revendication 1 dont la composition des amino-acides est d'environ 5,6% molaires d'acide aspartique, environ 8,1% molaires de thréonine, environ 12,9% molaires de sérine, environ 13,0% molaires d'acide glutamique, environ 11,6% molaires de proline, environ 7,7% molaires de glycine, environ 9,6% molaires d'alanine, environ 6,2% molaires de valine, environ 0,5% molaire de méthionine, environ 4,6% molaires d'isoleucine, environ 8,3% molaires de leucine, environ 1,6% molaire de tyrosine, environ 1,2% molaire de phénylalanine, environ 1,6% molaire d'histidine, environ 3,2% molaires de lysine, environ 4,0% molaires d'arginine et environ 0,3% molaire de tryptophane.

11. La glycoprotéine de mouton de la revendication 10 qui est une sialoglycoprotéine constituée d'environ 56,6% de restes d'hydrates de carbone en une composition d'environ 16,6 g d'acides sialiques, environ 9 g de galactose, environ 13,7 g de N-acétylgalactosamine, environ 14,4 g de N-acétylglucosamine et environ 1,7 g de mannose pour 100 g de glycoprotéine.

12. Un procédé pour le diagnostic de la mononucléose infectieuse qui comprend les stades de:

(a) fixation à de petites particules de latex ou de résine synthétique d'une glycoprotéine de l'une quelconque des revendications 6 à 11 ou préparée par un procédé de l'une quelconque des revendications 1 à 5;

(b) revêtement d'une surface plane avec des particules du stade (a);

(c) addition d'un échantillon dilué d'un liquide biologique à étudier provenant d'un patient soupçonné d'être atteint de mononucléose infectieuse, et rotation de la surface plane;

(d) observation du degré d'agglutination de l'échantillon; et

(e) comparaison du résultat du stade (d) avec le degré d'agglutination observé lorsqu'on remplace l'échantillon dans le stade (c) du mode opératoire décrit dans les stades (a) à (d) par un témoin contenant une quantité connue d'anticorps hétérophile de la mononucléose infectieuse.

13. Une glycoprotéine selon l'une quelconque des revendications 6 à 11 ou préparée selon un procédé selon l'une quelconque des revendications 1 à 5 contenant un marqueur choisi dans le groupe constitué par les radio-isotopes, les fluorogènes, les chromophores, les enzymes, les substances luminescentes et d'autres marqueurs connus utilisés dans les dosages immunologiques.

14. La glycoprotéine marquée de la revendication 13 dont le marqueur est [125]I.

15. Une opération de dosage radio-immunologique pour déterminer la quantité d'anticorps hétérophile de la mononucléose infectieuse humaine présente dans un échantillon de liquide à étudier dans lequel l'antigène correspondant audit anticorps comprend une glycoprotéine radiomarquée de la revendication 13 ou de la revendication 14.

16. Une opération selon la revendication 15 dans laquelle le dosage radio-immunologique est du type sandwich.

17. Une opération selon la revendication 15 dans laquelle le dosage radio-immunologique est un dosage par compétition.

18. Une opération de dosage radio-immunologique selon la revendication 17 dans laquelle la glycoprotéine est liée à une surface solide.

19. Une glycoprotéine érythrocytaire de cheval ou de mouton selon l'une quelconque des revendications 7 à 11 ou préparée par un procédé selon l'une quelconque des revendications 1 à 5 pour utilisation comme réactif stable et normalisable pour la numérotation des lymphocytes formant des rosettes.

20. Une glycoprotéine selon l'une quelconque des revendications 6 à 11 ou préparée par un procédé selon l'une quelconque des revendications 1 à 5 pour utilisation comme réactif de diagnostic utile dans la détection de la mononucléose infectieuse.

21. Un procédé pour l'inhibition de la formation de rosettes par les lymphocytes du sang humain qui comprend l'incubation desdits lymphocytes avec une glycoprotéine de cheval ou de mouton selon l'une quelconque des revendications 7 à 11 ou préparée par un procédé selon l'une quelconque des revendications 1 à 5.

22. Un procédé pour la surveillance ou le diagnostic de la mononucléose infectieuse humaine qui comprend l'utilisation d'une glycoprotéine selon l'une quelconque des revendications 6 à 11 ou préparée par un procédé selon l'une quelconque des revendications 1 à 5 comme inhibiteur de l'agglutination.